(19) Europäisches Patentamt
European Patent Office
Office européen des brevets

(11) **EP 3 377 140 B1**

(12) **EUROPÄISCHE PATENTSCHRIFT**

(45) Veröffentlichungstag und Bekanntmachung des Hinweises auf die Patenterteilung:
**07.10.2020 Patentblatt 2020/41**

(21) Anmeldenummer: **16798411.1**

(22) Anmeldetag: **17.11.2016**

(51) Int Cl.:
*A61M 1/16* (2006.01)      *A61M 1/34* (2006.01)
*A61M 1/36* (2006.01)

(86) Internationale Anmeldenummer:
**PCT/EP2016/001946**

(87) Internationale Veröffentlichungsnummer:
**WO 2017/084757 (26.05.2017 Gazette 2017/21)**

(54) **DIALYSEGERÄT MIT HEIZ- UND KÜHLMECHANISMUS**

DIALYSIS DEVICE COMPRISING HEATING AND COOLING MECHANISM

APPAREIL DE DIALYSE PRÉSENTANT UN MÉCANISME DE CHAUFFAGE ET DE REFROIDISSEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.11.2015 DE 102015014859**

(43) Veröffentlichungstag der Anmeldung:
**26.09.2018 Patentblatt 2018/39**

(73) Patentinhaber: **Fresenius Medical Care Deutschland GmbH**
**61352 Bad Homburg (DE)**

(72) Erfinder: **SPICKERMANN, Reiner**
**97535 Wasserlosen-Burghausen (DE)**

(74) Vertreter: **Herrmann, Uwe et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**EP-A1- 2 995 329**      **WO-A1-2015/074999**
**DE-A1-102008 062 424**  **JP-A- 2000 093 449**
**US-A- 6 156 007**

## Beschreibung

Die Erfindung betrifft ein Dialysegerät mit einem extrakorporalen Blutsystem, einem Dialysierflüssigkeitssystem, einem Dialysator und einer Steuereinheit.

Dialysegeräte gemäß dem Oberbegriff des Anspruchs 1 sind aus dem Stand der Technik bekannt. Derartige Geräte werden im Rahmen einer Dialysebehandlung dazu verwendet, Harnstoff und andere Stoffe aus dem Blut eines Patienten mit fehlender oder verminderter Nierenaktivität zu entfernen. Das zentrale Element eines Dialysegeräts ist der Dialysator, wobei es sich um eine Filtereinheit mit einer Blutkammer und einer Dialysierflüssigkeitskammer handelt, die durch eine semipermeable Membran getrennt sind. Die aus dem Blut zu entfernenden Stoffe treten im Dialysator durch die semipermeable Membran von der Blutkammer in die Dialysierflüssigkeitskammer über.

Beispielsweise ist aus der Druckschrift DE102008062424A1 ein Verfahren zur Verringerung der Blutgerinnung im Kreis eines Gerätes zum Ersatz der Nierenfunktion bekannt. Die Verringerung der Blutgerinnung im Kreis des Gerätes zum Ersatz der Nierenfunktion wird dadurch erreicht, dass vor dem Eintritt in das Gerät zum Ersatz der Nierenfunktion das Blut nach dem Verlassen des Körpers des Patienten auf eine Temperatur im Bereich von 10° bis 30°C abgekühlt wird, dass nach dem Durchgang des Blutes durch das Gerät zum Ersatz der Nierenfunktion das Blut auf eine zumindest der Körpertemperatur nahe Temperatur erwärmt wird und dass darauf das Blut in den Körper des Patienten zurückgeleitet wird.

Zudem ist aus der Patentschrift US6156007A ein Verfahren zur Behandlung eines Patienten mittels Hyperthermie bekannt, bei dem einem Patienten entnommenes Blut mittels einer Dialysemaschine zunächst auf eine Temperatur erwärmt wird, die oberhalb der Körpertemperatur liegt, und daraufhin nach Austritt aus dem Dialysator der Dialysemaschine nochmals erwärmt wird, wodurch eine Hyperthermie des Patienten erreicht wird. Aus der EP2995329 A1, die zum Stand der Technik gemäß Artikel 54(3) EPÜ gehört, ist eine Vorrichtung zur extrakorporalen Blutbehandlung mit einer Heizvorrichtung bekannt.

Um die Effizienz der Behandlung zu steigern, ist es wünschenswert, einen möglichst hohen Durchsatz der unerwünschten Stoffe von der Blutkammer in die Dialysierflüssigkeitskammer zu erreichen.

Vor diesem Hintergrund betrifft die Erfindung ein Dialysegerät mit einem extrakorporalen Blutsystem, einem Dialysierflüssigkeitssystem, einem Dialysator und einer Steuereinheit. Erfindungsgemäß weist das Dialysegerät einen Heizmechanismus zur Erwärmung des Blutes im extrakorporalen Blutsystem vor Eintritt in den Dialysator oder im Dialysator sowie einen Kühlmechanismus zur Abkühlung des Blutes im extrakorporalen Blutsystem nach Austritt aus dem Dialysator auf. Die Steuereinheit ist so ausgebildet, dass das Blut vor Eintritt in den Dialysator oder im Dialysator auf eine Dialysetemperatur erwärmt wird, die oberhalb der Körpertemperatur des Patienten liegt, und nach Austritt aus dem Dialysator wieder auf die Körpertemperatur des Patienten abgekühlt wird.

Die Erfindung macht sich die Erkenntnis zu Nutze, dass eine Erhöhung der Bluttemperatur im Dialysator einen positiven Effekt auf die Reinigungsleistung im Dialysator hat. Die Erhöhung der Bluttemperatur kann hierbei durch unterschiedliche Vorrichtungen außerhalb des Körpers erfolgen. Wesentlich für die Sicherheit des Patienten ist, dass das zurückfließende Blut wieder Körpertemperatur (mit einem gewissen Toleranzbereich) hat.

Die Temperaturerhöhung beeinflusst die Reinigungsleistung im Dialysator aufgrund mehrerer Effekte. Beispielsweise erhöht sich die Diffusion mit steigender Temperatur, während sich die Viskosität des Bluts verringert. Ferner wird bei erhöhter Temperatur das Gleichgewicht zwischen gebundenen Toxinen, z.B. Albumingebundenen Toxinen, die nicht membrangängig sind, und freien Toxinen, die membrangängig sind, in Richtung der freien Toxine verschoben. Diese Effekte addieren sich und führen insgesamt zu einer signifikant höheren Reinigungsleistung.

In einer Ausführungsform liegt die Dialysetemperatur zwischen 37°C und 46°C, vorzugsweise zwischen 40°C und 46°C und weiter vorzugsweise bei zwischen 42°C und 45°C. Bei Temperaturen jenseits der Grenze von etwa 46°C können Denaturierungen und andere unerwünschte Effekte im Blut einsetzen.

In einer Ausführungsform umfasst der Heizmechanismus eine vorlaufseitig des Dialysators im Dialysierflüssigkeitssystem angeordnete Heizvorrichtung. Die Steuereinheit ist in dieser Ausführungsform so ausgebildet, dass die Dialysierflüssigkeit vor Eintritt in den Dialysator auf eine Temperatur erhitzt wird, die größer oder wenigstens gleich der Dialysetemperatur ist. So kann das Blut durch Wärmetausch im Dialysator auf die Dialysetemperatur erwärmt werden. Der Grad der durch den Wärmetausch hervorgerufenen Erwärmung kann bei gegebener Bauart des Dialysators durch deine Regelung der Flüsse der Dialysierflüssigkeit und des Blutes im Dialysator sowie durch eine Einstellung der Temperatur der Dialysierflüssigkeit erfolgen.

In einer Ausführungsform umfasst das Dialysegerät ein Substitutionsflüssigkeitssystem, das eine vorlaufseitig des Dialysators in das extrakorporale Blutsystem mündende Predilutionsleitung und/oder eine rücklaufseitig des Dialysators in das extrakorporale Blutsystem mündende Postdilutionsleitung umfasst.

In einer Ausführungsform umfasst der Heizmechanismus eine vorlaufseitig der Mündung der Predilutionsleitung im Substitutionsflüssigkeitssystem angeordnete Heizvorrichtung. Die Steuereinheit ist in dieser Ausführungsform so ausgebildet, dass die Substitutionsflüssigkeit vor Eintritt in das extrakorporale Blutsystem durch die Predilutionsleitung auf eine Temperatur erhitzt wird, die größer oder wenigstens gleich der Dialysetemperatur ist. So kann das Blut durch die Zugabe einer warmen Substitutionsflüssigkeit vor Eintritt in den Dialysator auf die Dialysetemperatur erwärmt werden. Der Grad der Erwärmung kann durch deine Regelung der Flüsse der Substitutionsflüssigkeit und des Blutes sowie durch eine Einstellung der Temperatur der Substitutionsflüssigkeit erfolgen.

In einer Ausführungsform ist das Substitutionsflüssigkeitssystem in das Dialysierflüssigkeitssystem integriert und die Heizvorrichtungen für die Dialysierflüssigkeit und die Substitutionsflüssigkeit können einander entsprechen.

In einer Ausführungsform umfasst der Kühlmechanismus eine vorlaufseitig der Mündung der Postdilutionsleitung im Substitutionsflüssigkeitssystem angeordnete Kühlvorrichtung. Die Steuereinheit ist in dieser Ausführungsform so ausgebildet, dass die Substitutionsflüssigkeit vor Eintritt in das extrakorporale Blutsystem durch die Postdilutionsleitung auf eine Temperatur abgekühlt wird, die kleiner oder maximal gleich der Körpertemperatur ist.

In einer Ausführungsform umfasst der Kühlmechanismus eine vorlaufseitig der Heizvorrichtung im Substitutionsflüssigkeitssystem angeordnete Abzweigung für Substitutionsflüssigkeit, deren Temperatur kleiner oder maximal gleich der Körpertemperatur ist. Die Steuereinheit ist in dieser Ausführungsform so ausgebildet, dass die Substitutionsflüssigkeit vor Eintritt in die Heizvorrichtung abgezweigt und vor Eintritt in das extrakorporale Blutsystem durch die Postdilutionsleitung nicht bis auf Körpertemperatur erwärmt wird.

An Stelle der Abzweigung können auch separate Substitutionsflüssigkeitssysteme für Predilution und Postdilution vorliegen.

So kann das Blut durch die Zugabe einer kühlen Substitutionsflüssigkeit nach Austritt aus dem Dialysator wieder von der Dialysetemperatur auf Körpertemperatur abgekühlt werden. Der Grad der Abkühlung kann durch deine Regelung der Flüsse der Substitutionsflüssigkeit und des Blutes sowie durch eine Einstellung der Temperatur der Substitutionsflüssigkeit erfolgen.

In einer Ausführungsform umfasst der Heizmechanismus eine vorlaufseitig des Dialysators im Blutsystem angeordnete Heizvorrichtung. In einer Ausführungsform umfasst Kühlmechanismus eine rücklaufseitig des Dialysators im Blutsystem angeordnete Kühlvorrichtung. So kann eine direkte Erwärmung bzw. Abkühlung des Blutes erfolgen.

Im Wesentlichen ist die Erhöhung der Bluttemperatur durch indirekte Heizungen (bspw. Durchlauferhitzer), Einleitung erwärmter Flüssigkeit (warme Prädilution, vorstellbar ist auch erwärmtes Citrat bei CiCa Antikoagulation) oder durch Wärmetausch im Dialysator selbst (warmes Dialysat) möglich. Es ist auch die indirekte Kühlung des Bluts (Peltierelement, Kühlelement etc.) denkbar.

In einer Ausführungsform handelt es sich bei der Heizvorrichtung um einen an einer Leitung des jeweiligen Flüssigkeitssystems angeordneten Durchlauferhitzer. Bei der Kühlvorrichtung kann es sich um einen an einer Leitung des jeweiligen Flüssigkeitssystems angeordneten Durchlaufkühler handeln.

In einer Ausführungsform umfasst die Heizvorrichtung als Heizelement einen Wärmetauscher, beispielsweise einen Spiralwärmetauscher, ein Peltierelement und/oder einen Heizbeutel. Die Kühlvorrichtung kann als Kühlelement einen Wärmetauscher, beispielsweise einen Spiralwärmetauscher, ein Peltierelement und/oder einen Kühlbeutel umfassen.

In einer Ausführungsform weist das Dialysegerät ein Zugabesystem für eine Antikoagulationsmittelflüssigkeit wie beispielsweise eine Citratlösung oder eine Heparinlösung auf, das eine vorlaufseitig und/oder rücklaufseitig des Dialysators in das extrakorporale Blutsystem mündende Zugangsleitung umfasst. Anhand dieses Zugabesystems und der Temperierung der Antikoagulationsmittelflüssigkeit kann ebenfalls eine Temperaturkontrolle bzw. ein Beitrag zur Temperaturkontrolle erreicht werden, wie dies im Zusammenhang mit dem Substitutionsmittelflüssigkeitssystem erläutert wurde.

Zur Kontrolle der Temperatur aller Flüssigkeiten können an geeigneten Stellen im Dialysegerät Temperatursensoren angeordnet sein, die mit der Steuereinheit in Verbindung stehen. Beispielsweise kann im extrakorporalen Blutsystem ein Temperatursensor vor oder am Dialysator angeordnet sein, um die Temperatur des Blutes vor dem oder im Dialysator zu überwachen. Ferner kann im Blutsystem rücklaufseitig der Kühlvorrichtung oder Mündung ein Temperatursensor angeordnet sein, um die Temperatur des Blutes vor der Reinfusion an den Patienten zu überwachen. Des Weiteren kann im Dialysierflüssigkeitssystem ein Temperatursensor vor dem Dialysator angeordnet sein, um die Temperatur der Dialysierflüssigkeit vor dem Dialysator zu überwachen. Auch kann in der Prä- und/oder Postdilutionsleitung des Substitutionsflüssigkeitssystems ein Temperatursensor vor der Mündung angeordnet sein, um die Temperatur der Substitutionsflüssigkeit vor Eintritt in das extrakorporale Blutsystem zu überwachen.

Sowohl der Heizmechanismus als auch der Kühlmechanismus kann mehrere unterschiedliche der genannten entsprechenden Mechanismen kombinieren.

Vorzugsweise übersteigt die Temperatur der erwärmten Dialysierflüssigkeitslösung, Substitutionslösung oder Antikoagulationsmittellösung bzw. die Temperatur der am Blutsystem angeordneten Heizelemente eine Temperatur von 46°C nicht, um keine lokale Denaturierung von Blutbestandteilen bei der Zugabe bzw. an der Kontaktstelle zu verursachen.

Anhand des erfindungsgemäßen Dialysegeräts kann ein Dialyseverfahren durchgeführt werden, wobei das Blut vor Eintritt in den Dialysator oder im Dialysator auf die Dialysetemperatur erwärmt und nach Austritt aus dem Dialysator wieder auf die Körpertemperatur des Patienten abgekühlt wird.

Weitere Einzelheiten und Vorteile der Erfindung ergeben sich aus den nachfolgend anhand der Figuren dargestellten Ausführungsbeispielen. In den Figuren zeigen:

Figur 1:     eine modellierte Darstellung der Änderung des Diffusionskoeffizienten D in wässriger Lösung nach Stokes-Einstein-Gleichung;

Figur 2:     eine schematische Darstellung einer Ausführungsform eines erfindungsgemäßen Dialysegeräts;

Figur 3:     eine schematische Darstellung einer weiteren Ausführungsform eines erfindungsgemäßen Dialysegeräts;

Figur 4:     eine schematische Darstellung einer weiteren Ausführungsform eines erfindungsgemäßen Dialysegeräts; und

Figur 5:     eine schematische Darstellung einer weiteren Ausführungsform eines erfindungsgemäßen Dialysegeräts.

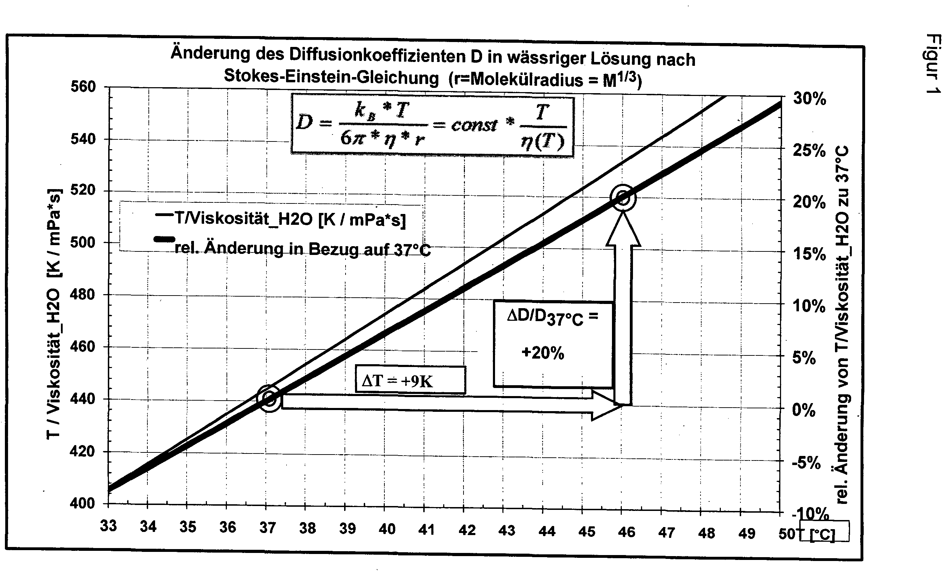

Eine modellierte Darstellung der Änderung des Diffusionskoeffizienten D in wässriger Lösung nach Stokes-Einstein-Gleichung ist in Figur 1 dargestellt. Die Stokes-Einstein-Gleichung beschreibt den Diffusionskoeffizienten in Abhängigkeit der Temperatur T, der Viskosität $\eta$ des Lösungsmittels und dem Radius r des diffundierenden Moleküls.

$$D = k_B \cdot T \, / \, 6 \cdot \pi \cdot \eta \cdot r = \text{const} \cdot T \, / \, \eta(T)$$

Die Viskosität des Wassers (Plasmas) ist ebenfalls temperaturabhängig. In der Darstellung gemäß Figur 1 ist daher das Verhältnis T/$\eta$ und dessen relative Änderung in Bezug auf 37°C als Funktion der Temperatur dargestellt. Bei einer Temperatur-Erhöhung von 37°C auf 46°C ($\Delta$T=+9K) ergibt sich danach eine Erhöhung der Diffusionskoeffizienten D um 20% für alle Moleküle.

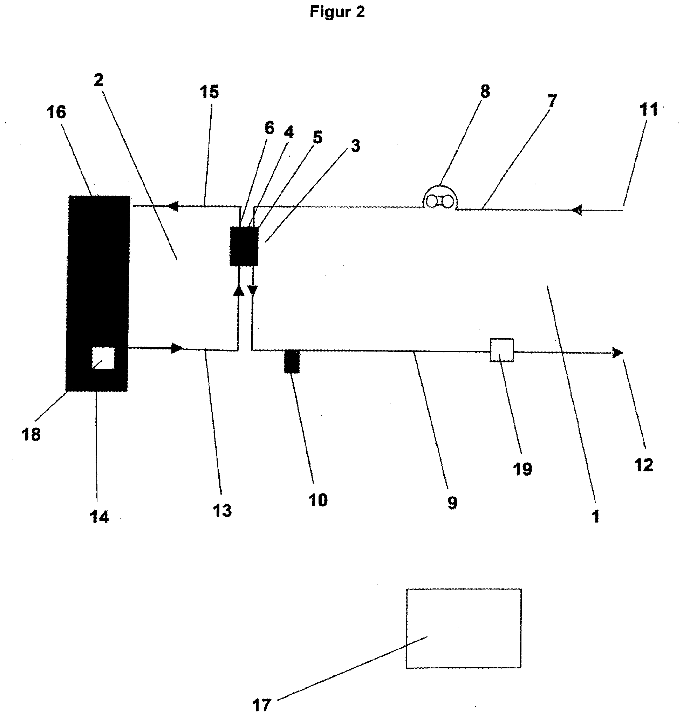

In Figur 2 ist eine erste Ausführungsform eines erfindungsgemäßen Dialysegerätes schematisch dargestellt.

Das Dialysegerät umfasst einen extrakorporalen Blutkreislauf 1 und einen Dialysierflüssigkeitskreislauf 2, welche an einem Dialysator 3 miteinander in Kontakt treten. Der Dialysator 3 umfasst eine semipermeable Membran 4, welche eine Blutkammer 5, die einen Teil des extrakorporalen Blutkreislaufs 1 bildet, und eine Dialysierflüssigkeitskammer 6, die einen Teil des Dialysierflüssigkeitskreislaufs 2 bildet, voneinander trennt. Die Flussrichtungen des Bluts und der Dialysierflüssigkeit in den unterschiedlichen Kammern 5 und 6 des Dialysators 3 sind gegenläufig. Die Flussrichtungen in den Kreisläufen sind in der Figur mit Pfeilen angedeutet.

In der arteriellen Blutleitung 7 befindet sich eine Blutpumpe 8 und in der venösen Blutleitung 9 befindet sich eine Tropfkammer 10. Der arterielle Port und der venöse Port 12 zum Anschluss des Patienten sind mit den Bezugzeichen 11 und 12 gekennzeichnet.

Die Vorlaufleitung 13 des Dialysierflüssigkeitskreislaufs 2 ist mit einer Dialysierflüssigkeitsquelle 14 verbunden. Bei der Quelle kann es sich beispielsweise um ein maschinenindividuelles Reservoir oder eine maschinenindividuelle kontinuierliche Mischeinheit handeln: Ferner ist denkbar, dass die Quelle 14 eine zentrale Versorgungseinheit eines Dialysezentrums darstellt. Die Rücklaufleitung 15 des Dialysierflüssigkeitskreislaufs 2 ist mit einem Ablauf 16 verbunden.

Das Dialysegerät weist ferner eine Steuereinheit 17 auf, welche unter anderem die Flussraten im extrakorporalen Blutkreislauf 1 und im Dialysierflüssigkeitskreislauf 2 anhand der Pumpe 8 und weiterer, in Figur 2 nicht dargestellter, aber den Fachmann bekannter Aktoren regelt.

Erfindungsgemäß weist das Dialysegerät einen Heizmechanismus zur Erwärmung des Blutes beim Durchlaufen des Dialysators 3 auf. In der gezeigten Ausführungsform umfasst der Heizmechanismus eine Heizvorrichtung 18, welche in der Quelle 14 bzw: in der Vorlaufleitung 13 angeordnet ist und die (in der Figur nicht dargestellt) mit der Steuereinheit 17 verbunden ist. Diese Heizvorrichtung 18 wird von der Steuereinheit 17 so angesteuert, dass Dialysierflüssigkeit, bevor sie in die Dialysierflüssigkeitskammer 6 des Dialysators 3 eintritt, auf eine Temperatur erhöht, die oberhalb der Körpertemperatur des Patienten liegt. Durch einen Wärmeaustausch an der semipermeablen Membran 4 wird das Blut beim Durchlaufen der Blutkammer 5 des Dialysators 3 fortlaufend erwärmt, bis es nahe dem venösen Auslass des Dialysators 3 eine Dialysetemperatur erreicht, welche vorzugsweise oberhalb von 40°C liegt. Dadurch wird zumindest in der venösen Hälfte des Dialysators 3 aufgrund der zuvor beschriebenen Effekte eine erhöhte Reinigungsleistung erreicht. Die Temperatur der in den Dialysator 3 eintretenden Dialysierflüssigkeit kann beispielsweise anhand eines in der Figur nicht dargestellten Temperatursensors überwacht werden, der in der Vorlaufleitung 13 des Dialysierflüssigkeitskreislaufs 2, vorzugsweise nahe dem Dialysator 3, befindet und ebenfalls mit der Steuereinheit 17 verbunden ist.

Um das Blut, welches im Dialysator 3 auf eine Dialysetemperatur, die oberhalb der Körpertemperatur des Patienten liegt, erwärmt wurde, vor Reinfusion in den Patienten am venösen Port 12 wieder auf Körpertemperatur zu kühlen, weist das Dialysegerät ferner einen Kühlmechanismus auf, die einen Wärmetauscher 19 in der venösen Leitung 9 umfasst. Der Wärmetauscher 19 kann beispielsweise als Spiralwärmetauscher ausgebildet sein, wobei das Blut mit einer Kühlflüssigkeit in wärmeleitenden Kontakt gebracht wird, die eine Temperatur unterhalb der Körpertemperatur aufweist. Der Wärmetauscher bzw. die Fluidpumpe für die Kühlflüssigkeit steht ebenfalls mit der Steuereinheit 17 in Verbindung. Zur Überwachung der Bluttemperatur vor Reinfusion am venösen Port 12 kann in der venösen Leitung 9, vorzugsweise nahe dem venösen Port 12 und jedenfalls zwischen dem Wärmetauscher 19 und dem venösen Port 12, ein in der Figur nicht dargestellter Temperatursensor vorhanden sein, welcher ebenfalls mit der Steuereinheit 17 verbunden ist.

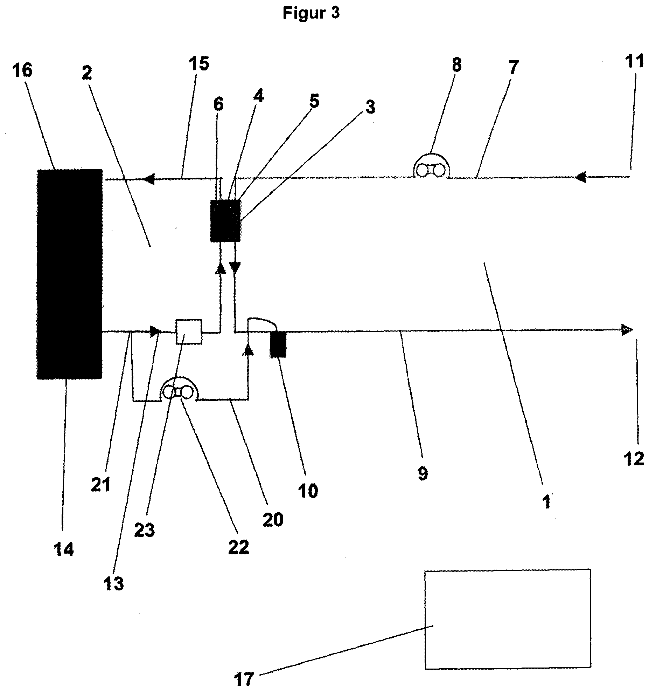

In Figur 3 wird eine weitere Ausführungsform eines erfindungsgemäßen Dialysegerätes gezeigt, wobei gleiche Teile mit gleichen Bezugszeichen versehen sind.

Dabei umfasst der Kühlmechanismus eine Postdilutionsleitung 20, die an einem Punkt 21 von der Vorlaufleitung 13 abzweigt. Innerhalb der Postdilutionsleitung ist eine Flüssigkeitspumpe 22 angeordnet. Die Postdilutionsleitung 20 mündet an der Tropfkammer 10 in die venöse Leitung 9.

In dieser Ausführungsform ist zwischen dem Verzweigungspunkt 21 und dem Dialysator 3 in der Vorlaufleitung 13 eine Heizvorrichtung 23 vorgesehen. So ist es möglich, die Temperatur der Dialysierflüssigkeit, welche dem Dialysator 3 zugeführt wird, nach Abzweigung der Substitutionslösung für die Postdilution noch zu erhöhen. Insoweit kann vorgesehen sein, dass die Dialysierflüssigkeit während der Bereitstellung in der Quelle 14 nicht oder zumindest nicht bis auf Körpertemperatur erwärmt wird und in diesem noch kühlen Zustand an der Verzweigungsstelle 21 in die Postdilutionsleitung 20 abgezweigt wird. Erst zwischen der Verzweigungsstelle 21 und dem Dialysator 3 erfolgt anhand der Heizvorrichtung 23 eine Erhöhung der Temperatur auf über Körpertemperatur, sodass sich die bereits im Zusammenhang mit der Ausführungsform gemäß Figur 1 dargestellten Erwärmungseffekte des Blutes im Dialysator 3 einstellen.

Die Heizvorrichtung 23 und die Pumpe 22 sind mit der Steuereinheit 17 verbunden.

Gegenüber der Vorrichtung gemäß Figur 1 erfolgt die Kühlung des Blutes nach Verlassen des Dialysators 3 jedoch nicht durch eine zusätzliche Kühleinheit 19 sondern durch die Zugabe von Substitutionslösung, welche eine Temperatur hat, die unterhalb der Körpertemperatur liegt. Der Vorteil dieser Lösung ist, dass kein Heiz- und/oder Kühlelement am extrakorporalen Blutkreislauf 1 notwendig ist.

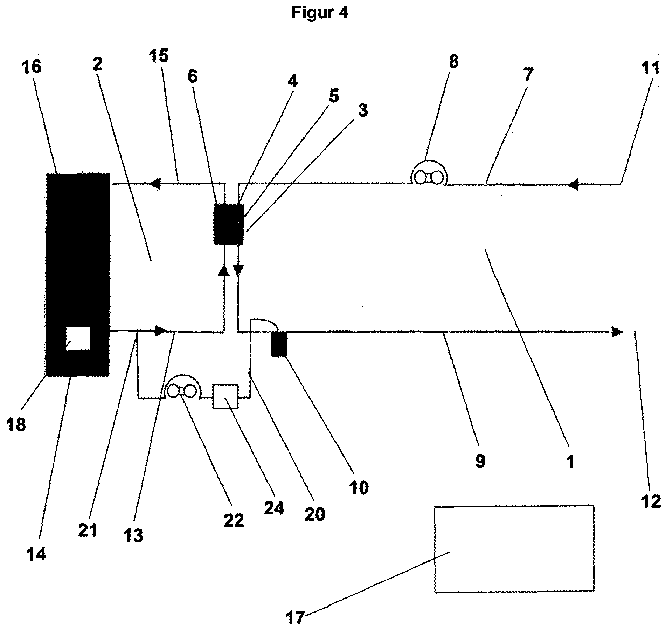

In Figur 4 wird eine weitere Ausführungsform der Erfindung gezeigt, wobei gleiche Teile wiederum mit identischen Bezugszeichen gekennzeichnet sind. Diese Ausführungsform ist der Ausführungsform gemäß Figur 3 ähnlich, mit dem einzigen Unterschied, dass statt der Heizvorrichtung 23 in der Vorlaufleitung 13 eine Kühlvorrichtung 24 in der Postdilutionsleitung 20 angeordnet ist. So wird die Dialysierflüssigkeit, wie dies auch in der Ausführungsform gemäß Figur 2 der Fall war, bereits während der Bereitstellung auf eine Temperatur oberhalb der Körpertemperatur erhöht und im Verzweigungspunkt 21 auch mit erhöhter Temperatur in die Postdilutionsleitung 20 abgezweigt. Die Dialysier- bzw. Substitutionsflüssigkeit wird anschließend in der Postdilutionsleitung 20 von der Kühlvorrichtung 24 gekühlt, bevor sie in die venöse Leitung 9 des extrakorporalen Blutkreislaufs 1 eingeleitet wird. Diese Ausführungsform hat gegenüber der Ausführungsform gemäß Figur 3 den Vorteil, dass anhand der selektiven Kühlung der Substitutionsflüssigkeit in der Postdilutionsleitung 20 eine verbesserte Regelbarkeit der Kühlleistung vorliegt.

Die Kühlvorrichtung 24, die Heizvorrichtung 18 und die Pumpe 22 sind mit der Steuereinheit 17 verbunden.

In den Ausführungsformen gemäß Figuren 3 und 4 kann in der Postdilutionsleitung 20 vorzugsweise nahe der Tropfkammer 10 ein Temperatursensor vorhanden sein, um die Temperatur der in die venöse Leitung 9 zugegebenen Substitutionslösung bestimmen zu können. Dieser Temperatursensor ist wiederum mit der Steuereinheit 17 verbunden.

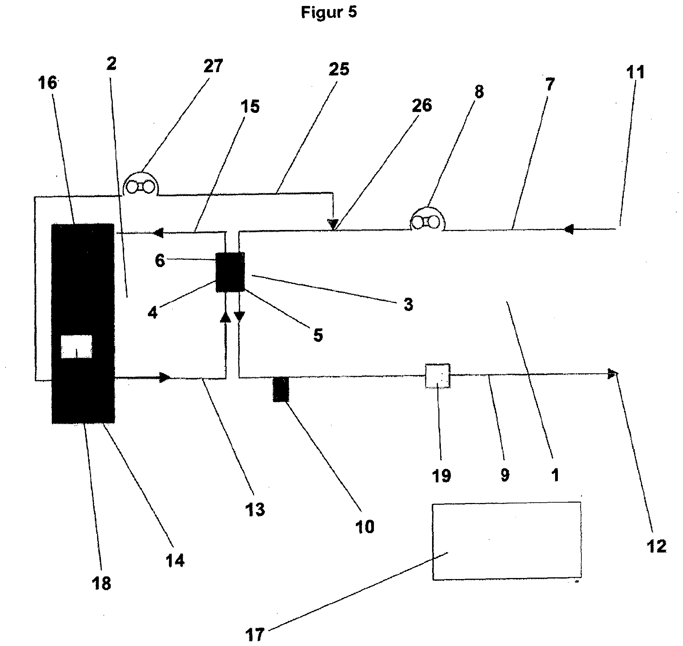

Figur 5 zeigt eine weitere Ausführungsform eines erfindungsgemäßen Dialysegerätes, wobei der Unterschied gegenüber der Ausführungsform gemäß Figur 2 darin besteht, dass eine Predilutionsleitung 25 vorgesehen ist, anhand der Substitutionslösung aus der Quelle 14 abgezweigt und an einer Mischstelle 26 zwischen der Pumpe 8 und dem Dialysator 3 in die arterielle Leitung 7 eingemischt wird.

In der Predilutionsleitung 25 befindet sich eine Pumpe 27. In dieser Ausführungsform besteht die Möglichkeit, in der Quelle 14 auf eine Temperatur oberhalb der Körpertemperatur des Patienten erwärmte Substitutionslösung in die arterielle Leitung 7 einzuleiten und somit das Blut bereits vor Eintritt in den Dialysator 3 auf eine Dialysetemperatur von beispielsweise größer 40°C zu erwärmen. Insoweit kann auf diese Weise die Behandlungseffizienz über den gesamten Dialysator gesteigert werden. Eine weitere Erwärmung durch die Verwendung einer über Körpertemperatur erwärmten Dialysierflüssigkeit auf der Dialysierflüssigkeitsseite 6 des Dialysators 3 ist zusätzlich möglich.

Vorzugsweise in dieser Ausführungsform vorgesehen, in der Predilutionsleitung 25 nahe der Einspritzstelle 26 einen Temperatursensor vorzusehen und diesen mit der Steuereinheit 17 zu verbinden, um die Temperatur der eingespritzten Substitutionslösung überwachen zu können. Die Pumpe 27 steht ebenfalls mit der Steuereinheit 17 in Verbindung.

**Patentansprüche**

1. Dialysegerät mit einem extrakorporalen Blutsystem, einem Dialysierflüssigkeitssystem, einem Dialysator (3) und einer Steuereinheit (17),
   **dadurch gekennzeichnet,**
   **dass** das Dialysegerät einen Heizmechanismus zur Erwärmung des Blutes im extrakorporalen Blutsystem vor Eintritt in den Dialysator (3) oder im Dialysator (3) sowie einen Kühlmechanismus zur Abkühlung des Blutes im extrakorporalen Blutsystem nach Austritt aus dem Dialysator (3) aufweist, und dass die Steuereinheit (17) so ausgebildet ist, dass das Blut vor Eintritt in den Dialysator (3) oder im Dialysator (3) auf eine Dialysetemperatur erwärmt wird, die oberhalb der Körpertemperatur des Patienten liegt, und nach Austritt aus dem Dialysator (3) wieder auf die Körpertemperatur des Patienten abgekühlt wird.

2. Dialysegerät nach Anspruch 1, **dadurch gekennzeichnet, dass** die Dialysetemperatur zwischen 37°C und 46°C, vorzugsweise zwischen 40°C und 46°C und weiter vorzugsweise zwischen 42°C und 45°C liegt.

3. Dialysegerät nach einem der vorhergehenden Ansprüche, **dadurch gekennzeichnet, dass** der Heizmechanismus eine vorlaufseitig des Dialysators (3) im Dialysierflüssigkeitssystem angeordnete Heizvorrichtung (18) umfasst und dass die Steuereinheit (17) so ausgebildet ist, dass die Dialysierflüssigkeit vor Eintritt in den Dialysator (3) auf eine Temperatur erhitzt wird, die größer oder wenigstens gleich der Dialysetemperatur ist.

4. Dialysegerät nach einem der vorhergehenden Ansprüche, **dadurch gekennzeichnet, dass** das Dialysegerät ferner ein Substitutionsflüssigkeitssystem aufweist, das eine vorlaufseitig des Dialysators (3) in das extrakorporale Blutsystem mündende Predilutionsleitung (25) und/oder eine rücklaufseitig des Dialysators (3) in das extrakorporale Blutsystem mündende Postdilutionsleitung (20) umfasst.

5. Dialysegerät nach Anspruch 4, **dadurch gekennzeichnet, dass** der Heizmechanismus eine vorlaufseitig der Mündung der Predilutionsleitung (25) im Substitutionsflüssigkeitssystem angeordnete Heizvorrichtung (18) umfasst und dass die Steuereinheit (17) so ausgebildet ist, dass die Substitutionsflüssigkeit vor Eintritt in das extrakorporale Blutsystem durch die Predilutionsleitung (25) auf eine Temperatur erhitzt wird, die größer oder wenigstens gleich der Dialysetemperatur ist.

6. Dialysegerät nach Anspruch 5, **dadurch gekennzeichnet, dass** der Kühlmechanismus eine vorlaufseitig der Mündung der Postdilutionsleitung (20) im Substitutionsflüssigkeitssystem angeordnete Kühlvorrichtung (24) umfasst und dass die Steuereinheit (17) so ausgebildet ist, dass die Substitutionsflüssigkeit vor Eintritt in das extrakorporale Blutsystem durch die Postdilutionsleitung (20) auf eine Temperatur abgekühlt wird, die kleiner oder maximal gleich der Körpertemperatur ist.

7. Dialysegerät nach Anspruch 5, **dadurch gekennzeichnet, dass** der Kühlmechanismus eine vorlaufseitig der Heizvorrichtung (18) im Substitutionsflüssigkeitssystem angeordnete Abzweigung für Substitutionsflüssigkeit aufweist, deren Temperatur kleiner oder maximal gleich der Körpertemperatur ist, und dass die Steuereinheit (17) so ausgebildet ist, dass die Substitutionsflüssigkeit vor Eintritt in die Heizvorrichtung (18) abgezweigt und vor Eintritt in das extrakorporale Blutsystem durch die Postdilutionsleitung (20) nicht bis auf Körpertemperatur erwärmt wird.

8. Dialysegerät nach einem der vorhergehenden Ansprüche, **dadurch gekennzeichnet, dass** der Heizmechanismus eine vorlaufseitig des Dialysators (3) im Blutsystem angeordnete Heizvorrichtung (23) umfasst und/oder dass der Kühlmechanismus eine rücklaufseitig des Dialysators (3) im Blutsystem angeordnete Kühlvorrichtung (24) umfasst.

9. Dialysegerät nach einem der Ansprüche 3 bis 8, **dadurch gekennzeichnet, dass** es sich bei der Heizvorrichtung (18, 23) um einen an einer Leitung des jeweiligen Flüssigkeitssystems angeordneten Durchlauferhitzer handelt und/oder dass die Heizvorrichtung (18, 23) als Heizelement einen Wärmetauscher (19), beispielsweise einen Spiralwärmetauscher, ein Peltierelement und/oder einen Heizbeutel umfasst.

10. Dialysegerät nach einem der Ansprüche 3 bis 9, **dadurch gekennzeichnet, dass** es sich bei der Kühlvorrichtung (24) um einen an einer Leitung des jeweiligen Flüssigkeitssystems angeordneten Durchlaufkühler handelt und/oder dass die Kühlvorrichtung (24) als Kühlelement einen Wärmetauscher (19), beispielsweise einen Spiralwärmetauscher, ein Peltierelement und/oder einen Kühlbeutel umfasst.

**Claims**

1. A dialysis device comprising an extracorporeal blood system, a dialyzing fluid system, a dialyzer (3) and a control unit (17),
**characterized in that**
the dialysis device has a heating mechanism for heating the blood in the extracorporeal blood system before entry into the dialyzer or in the dialyzer as well as a cooling mechanism for cooling the blood in the extracorporeal blood system after exiting the dialyzer (3); and **in that** the control unit (17) is configured such that the blood is heated before entry into the dialyzer (3) or in the dialyzer (3) to a dialysis temperature which is above the body temperature of the patient and is cooled back to the body temperature of the patient after exiting the dialyzer(3).

**2.** A dialysis device in accordance with claim 1, **characterized in that** the dialysis temperature is between 37°C and 46°C, preferably between 40°C and 46°C, and further preferably between 42°C and 45°C.

**3.** A dialysis device in accordance with one of the preceding claims, **characterized in that** the heating mechanism comprises a heating apparatus (18) arranged at the feed side of the dialyzer (3) in the dialyzing fluid system; and **in that** the control unit (17) is configured such that the dialyzing fluid is heated before entry into the dialyzer (3) to a temperature which is larger than or at least equal to the dialysis temperature.

**4.** A dialysis device in accordance with one of the preceding claims, **characterized in that** the dialysis device furthermore has a substitution fluid system which comprises a pre-dilution line (25) opening at the feed side of the dialyzer (3) into the extracorporeal blood system and/or a post-dilution line (20) opening at the return side of the dialyzer (3) into the extracorporeal blood system.

**5.** A dialysis device in accordance with claim 4, **characterized in that** the heating mechanism comprises a heating apparatus (18) arranged at the feed side of the opening of the pre-dilution line (25) in the substitution fluid system; and **in that** the control unit (17) is configured such that the substitution fluid is heated before entry into the extracorporeal blood system through the pre-dilution line (25) to a temperature which is larger than or at least equal to the dialysis temperature.

**6.** A dialysis device in accordance with claim 5, **characterized in that** the cooling mechanism comprises a cooling apparatus (24) arranged at the feed side of the opening of the post-dilution line (20) in the substitution fluid system; and **in that** the control unit (17) is configured such that the substitution fluid is cooled before entry into the extracorporeal blood system through the post-dilution line (20) to a temperature which is less than or at a maximum equal to body temperature.

**7.** A dialysis device in accordance with claim 5, **characterized in that** the cooling mechanism has a branch for substitution fluid arranged at the feed side of the heating apparatus (18) in the substitution fluid system, the temperature of the substitution fluid being less than or at a maximum equal to body temperature; and **in that** the control unit (17) is configured such that the substitution fluid is branched off before entry into the heating apparatus (18) and is not heated up to body temperature through the post-dilution line (20) before entry into the extracorporeal blood system.

**8.** A dialysis device in accordance with one of the preceding claims, **characterized in that** the heating mechanism comprises a heating apparatus (23) arranged at the feed side of the dialyzer (3) in the blood system; and/or **in that** the cooling mechanism comprises a cooling apparatus (24) arranged at the return side of the dialyzer (3) in the blood system.

**9.** A dialysis device in accordance with one of the claims 3 to 8, **characterized in that** the heating apparatus (18, 23) is a flow heater arranged at a line of the respective fluid system; and/or **in that** the heating apparatus (18, 23) comprises a heat exchanger (19), for example a spiral heat exchanger, a Peltier element and/or a heating pack, as a heating element.

**10.** A dialysis device in accordance with one of the claims 3 to 9, **characterized in that** the cooling apparatus (24) is a flow cooler arranged at a line of the respective fluid system; and/or **in that** the cooling apparatus (24) comprises a heat exchanger (19), for example a spiral heat exchanger, a Peltier element and/or a cooling pack, as a cooling element.


**Revendications**

**1.** Appareil de dialyse comprenant un système de sang extracorporel, un système de fluide de dialyse, un dialyseur (3) et une unité de commande (17), **caractérisé en ce que**
l'appareil de dialyse comporte un mécanisme de chauffage pour chauffer le sang dans le système de sang extracorporel avant l'entrée dans le dialyseur (3) ou dans le dialyseur (3) ainsi qu'un mécanisme de refroidissement pour refroidir le sang dans le système de sang extracorporel après la sortie hors du dialyseur (3), et **en ce que** l'unité de commande (17) est conçue de telle manière que le sang est chauffé à une température de dialyse avant l'entrée dans le dialyseur (3) ou dans le dialyseur (3), ladite température de dialyse étant supérieure à la température corporelle du patient, et est de nouveau refroidi à la température corporelle du patient après la sortie hors du dialyseur (3).

**2.** Appareil de dialyse selon la revendication 1, **caractérisé en ce que** la température de dialyse est comprise entre 37 °C et 46 °C, de préférence entre 40 °C et 46 °C et plus préférentiellement entre 42 °C et 45 °C.

**3.** Appareil de dialyse selon l'une des revendications précédentes, **caractérisé en ce que** le mécanisme de chauffage comprend un dispositif de chauffage (18) disposé en amont du dialyseur (3) dans le système de fluide de dialyse et **en ce que** l'unité de commande (17) est conçue de telle manière que le fluide de dialyse est chauffé à une température qui est supérieure ou au moins égale à la température de dialyse avant l'entrée dans le dialyseur (3).

4. Appareil de dialyse selon l'une des revendications précédentes, **caractérisé en ce que** l'appareil de dialyse comporte en outre un système de fluide de substitution, qui comprend une ligne de prédilution (25) débouchant dans le système de sang extracorporel en amont du dialyseur (3) et/ou une ligne de postdilution (20) débouchant dans le système de sang extracorporel en aval du dialyseur (3)

5. Appareil de dialyse selon la revendication 4, **caractérisé en ce que** le mécanisme de chauffage comprend un dispositif de chauffage (18) disposé dans le système de fluide de substitution en amont de l'embouchure de la ligne de prédilution (25) et **en ce que** l'unité de commande (17) est conçue de telle manière que le fluide de substitution est chauffé à une température qui est supérieure ou au moins égale à la température de dialyse par la ligne de prédilution (25) avant l'entrée dans le système de sang extracorporel.

6. Appareil de dialyse selon la revendication 5, **caractérisé en ce que** le mécanisme de refroidissement comprend un dispositif de refroidissement (24) disposé dans le système de fluide de substitution en amont de l'embouchure de la ligne de postdilution (20) et **en ce que** l'unité de commande (17) est conçue de telle manière que le liquide de substitution est refroidi à une température qui est inférieure ou au maximum égale à la température corporelle par la ligne de postdilution (20) avant l'entrée dans le système de sang extracorporel.

7. Appareil de dialyse selon la revendication 5, **caractérisé en ce que** le mécanisme de refroidissement comporte, disposée dans le système de fluide de substitution en amont du dispositif de chauffage (18), une ramification pour fluide de substitution, dont la température est inférieure ou au maximum égale à la température corporelle, et **en ce que** l'unité de commande (17) est conçue de telle manière que le fluide de substitution est séparé avant l'entrée dans le dispositif de chauffage (18) et n'est pas chauffé jusqu'à la température corporelle par la ligne de postdilution (20) avant l'entrée dans le système de sang extracorporel.

8. Appareil de dialyse selon l'une des revendications précédentes, **caractérisé en ce que** le mécanisme de chauffage comprend un dispositif de chauffage (23) disposé dans le système de sang en amont du dialyseur (3) et/ou **en ce que** le mécanisme de refroidissement comprend un dispositif de refroidissement (24) disposé dans le système de sang en aval du dialyseur (3).

9. Appareil de dialyse selon l'une des revendications 3 à 8, **caractérisé en ce que** le dispositif de chauffage (18, 23) est un réchauffeur à flux continu disposé sur une ligne du système de fluide respectif et/ou **en ce que** le dispositif de chauffage (18, 23) comprend, en guise d'élément chauffant, un échangeur de chaleur (19), par exemple un échangeur de chaleur en spirale, un élément Peltier et/ou une poche chauffante.

10. Appareil de dialyse selon l'une des revendications 3 à 9, **caractérisé en ce que** le dispositif de refroidissement (24) est un refroidisseur à flux continu disposé sur une ligne du système de fluide respectif et/ou **en ce que** le dispositif de refroidissement (24) comprend, en guise d'élément de refroidissement, un échangeur de chaleur (19), par exemple un échangeur de chaleur en spirale, un élément Peltier et/ou une poche de refroidissement.

EP 3 377 140 B1

Figur 1

$$D = \frac{k_B * T}{6\pi * \eta * r} = const * \frac{T}{\eta(T)}$$

Änderung des Diffusionkoeffizienten D in wässriger Lösung nach Stokes-Einstein-Gleichung (r=Molekülradius = M$^{1/3}$)

— T/Viskosität_H2O [K / mPa*s]
— rel. Änderung in Bezug auf 37°C

ΔT = +9K

ΔD/D$_{37°C}$ = +20%

rel. Änderung von T/Viskosität_H2O zu 37°C

30%    25%    20%    15%    10%    5%    0%    -5%    -10%

[°C]

50  49  48  47  46  45  44  43  42  41  40  39  38  37  36  35  34  33

560    540    520    500    480    460    440    420    400

T / Viskosität_H2O [K / mPa*s]

**Figur 2**

Figur 3

Figur 4

**Figur 5**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008062424 A1
- US 6156007 A
- EP 2995329 A1